# EUROPEAN PATENT APPLICATION

(11) **EP 4 414 382 A1**
(43) Date of publication of application: **14.08.2024**
(21) Application number: 23305163.0
(22) Date of filing: 07.02.2023
(51) Int. Cl.: C07K 14/705, G01N 33/00, A01K 67/033, A01N 63/00

(54) **GENETICALLY MODIFIED FLIES USED FOR THE DETECTION OF PHEROMONES**

(71) Applicant: Université de Lorraine, 54052 Nancy Cedex (FR); Centre National de la Recherche Scientifique, 75016 Paris (FR); Institut national de recherche pour l'agriculture, l'alimentation et l'environnement, 75338 Paris Cedex 07 (FR); SORBONNE UNIVERSITE, 75006 Paris (FR); Université Paris-Est Créteil Val de Marne, 94010 Creteil Cedex (FR); Institut de Recherche pour le Développement, 13572 Marseille Cedex 02 (FR)
(72) Inventor: MARTINEZ, Dominique, 13006 MARSEILLE (FR); LUCAS, Philippe, 78960 VOISINS LE BRETONNEUX (FR); JOUAITI, Mélanie, Waterloo,CANADA, N2L3G5 (CA); CHATTERJEE, Abhishek, 78530 BUC (FR)
(74) Representative: Gevers & Orès

(57) **Abstract**

The present invention concerns the use of genetically modified flies for the detection of pheromones, methods using said flies and biosensors comprising the same.

## Description

The present invention concerns the use of genetically modified flies for the detection of pheromones, methods using said flies and biosensors comprising the same.

Insect pests directly or indirectly destroy 1/3 of annual crops, which represents major economic losses: hundreds of million €/year in Europe for the red palm weevil and 2-5 billion €/year for moths, such as the fall armyworm. In addition, the situation is worsening due to global warming which is accelerating the life cycle (number of generations per year) of harmful insects. Early detection of insect pests thus became a major challenge for optimal action before infestation.

Many pest species are nocturnal while their larvae remain hidden underground or deep within plant tissue - that makes visual detection nearly impossible, and therefore, necessitates alternative modes of monitoring. As insects communicate with species-specific pheromones, these compounds constitute signals indicating the presence of a given insect species. Current detection methods involve attracting insects into traps by broadcasting specific pheromones - the number of captures indicating population levels. However, such methods have many drawbacks since they require frequent human intervention (counting/identification of captures), a broadcast attractive pheromone that is difficult to maintain, the need to adapt the traps to the biology of each pest, and the risk of attracting insects from neighboring plots. The detection of insect pheromones can be an alternative for insect monitoring, but it remains a major challenge due to the low quantities emitted (ng-µg/day/insect). Indeed, current artificial sensors (e.g. gas sensors based on metal oxide) cannot detect pheromones at these doses.

Thus there is a need for some efficient means allowing the early detection of insect pests.

The present invention is believed to meet such a need by providing biosensors engineered from insect pheromone receptors (PRs), which allow unmatched olfactory performances.

The Inventors have developed an innovative pheromone detection solution by using genetically modified flies which express heterologous PRs from insect pests for the detection of pheromones in the environment. This unconventional approach has many advantages since the so-called "sentinel" flies constitute biosensors which are ultra-sensitive and selective to the pest pheromone, unlike wild flies which can be used as a natural control. The presence of the pest pheromone produces changes in the antenna electrical signal and the behavior of the sentinel flies and these changes can be detected in real time by an artificial intelligence (Al) algorithm. This approach is completely original and breaks with the classic approach in artificial olfaction where "electronic noses" have low sensitivity and poor selectivity.

In a main aspect, the present invention thus concerns the use of a genetically modified fly expressing a heterologous pheromone receptor for detecting the presence or absence of the pheromone in an environment, the presence or absence of the pheromone being detected by measuring the response of the genetically modified fly exposed to the environment.

As used herein, the term "fly" refers to any insect of the order Diptera, preferably of the suborder Brachycera, more preferably of the genus *Drosophila.* Although *Drosophila melanogaster* is preferred, it is contemplated that any fly of the genus *Drosophila* may be used in the present invention.

As used herein, the expression "genetically modified fly" refers to a fly that has had extra genetic material inserted into its genome. In practicing the present invention, many conventional techniques in molecular biology and recombinant DNA can be used. These techniques are well known to those skilled in the art and are explained, for example, in Montagn6 et al., Eur. J. Neurosci., 36(5):2588-96, 2012.

As used herein, the term "pheromone" designates any chemical substance produced and released into the environment by an animal, especially an insect, affecting the behavior or physiology of others of its species. Pheromones include, but are not limited to, volatile sex pheromones and aggregation pheromones. For example, some pheromones of interest can be 4-methylnonan-5-ol (= ferrugineol) produced by *Rhynchophorus ferrugineus,* (Z)-9-tetradecenyl acetate (Z9-14:Ac) produced by *Spodoptera frugiperda* and (Z,E)-9,11-tetradecadienyl acetate (Z9,E11-14:Ac) produced by *Spodoptera littoralis.*

In the case where the pheromone is a substance that comprises more than one compounds, the expression "pheromone" preferably designates the majority compound of the substance.

As used herein, the expression "heterologous pheromone receptor" refers to a pheromone receptor that is not naturally expressed in the fly.

As used herein, the term "environment" refers to the surroundings (e.g. air, land and water) in which animals, in particular insects, live or operate. Preferably, the invention is used in an agricultural site, such as an agricultural field.

In an embodiment, the invention relates to the used as defined above, wherein the pheromone is from an insect pest.

As used herein the expression "insect pest" means any invertebrate, in particular any insect, injurious to plants or plant products.

In an embodiment, the invention relates to the use as defined above, wherein the insect pest is chosen from the group comprising coleoptera, diptera, hemiptera, hymenoptera, lepidoptera, neuroptera, orthoptera or thysanopetera.

In an embodiment, the invention relates to the use as defined above, wherein the insect pest is chosen from the group comprising the genera *Spodoptera* and *Rhynchophorus.*

In an embodiment, the invention relates to the use as defined above, wherein the insect pest is chosen from the group comprising *Spodoptera frugiperda, Spodoptera littoralis* or *Rhynchophorus ferrugineus.*

In an embodiment, the insect pest is *Spodoptera frugiperda* (also called "Fall armyworm").

In an embodiment, the insect pest is *Spodoptera littoralis* (also called "African cotton leafworm", "Egyptian cotton leafworm" or "Mediterranean brocade").

In an embodiment, the insect pest is *Rhynchophorus ferrugineus* (also called "Red palm weevil", "Asian palm weevil" or "Sago palm weevil").

In an embodiment, the invention relates to the use as defined above, wherein the heterologous pheromone receptor is expressed in the majority of the fly's olfactory receptor neurons.

As used herein, the expression "the majority of the fly's olfactory receptor neurons" means that the heterologous pheromone receptor is expressed in more than 50% of the fly's olfactory receptor neurons. The genetically modified fly then becomes ultra-sensitive to the pest pheromone, unlike a wild fly which is not.

In particular, the heterologous pheromone receptor is expressed in >50%, ≥60%, ≥70%, ≥80%, ≥90% or 100% of the fly's olfactory receptor neurons. Preferably, it is expressed in at least 80% of the fly's olfactory receptor neurons. More preferably, it is expressed in all of the fly's olfactory receptor neurons.

The heterologous pheromone receptor can be expressed in addition to endogenous olfactory receptors.

However, the heterologous pheromone receptor can also be expressed in place of an endogenous fly's receptor. In such an embodiment, the heterologous pheromone receptor is expressed in a fly's olfactory receptor neuron in place of its native olfactory receptor.

Techniques allowing the production of transgenic flies are known to those skilled in the art and are explained, for example, in Montagn6 et al., Eur. J. Neurosci., 36(5):2588-96, 2012.

In an embodiment, the invention relates to the use as defined above, wherein the response of the genetically modified fly is an electrical signal in its antenna.

Indeed, during stimulation with pheromone, voltage fluctuations occur between the fly's antenna and another fly's tissue (e.g. the eye) caused by electrical depolarizations of the olfactory receptor neurons in the antenna.

The electrical signal is preferably recorded by Electroantennography (EAG).

In an embodiment, the invention relates to the use as defined above, wherein the response of the genetically modified fly is a change in its locomotor activity.

Indeed, the detection of the pheromone is also associated to behavior modifications of the fly. It is thus possible to detect the presence of the targeted pheromone by detecting a modification in the locomotor activity of the fly.

For example, such a change in fly's locomotor activity can be measured by infrared beam-crossing or video tracking.

More specifically, such a change in fly's locomotor activity can be measured as the average number of infrared beam-crossings per minute by the fly.

In a preferred embodiment, the response of the genetically modified fly (e.g. an electrical signal or a change in its locomotor activity) is compared to a reference response (e.g. another electrical signal or another locomotor activity).

In a more preferred embodiment, the reference response is the response of a wild type fly exposed to the same environment.

Indeed, one advantage of the invention is that it is possible to determine whether the response of the genetically modified fly is relevant or not by comparing it to a natural control, i.e. the response of the wild-type fly.

In an embodiment, the invention relates to the use as defined above, wherein the response of the genetically modified fly is compared to the response of a wild type fly exposed to the environment,
- a difference between the response of the genetically modified fly and the response of the wild type fly being indicative of the presence of the pheromone in the environment, and
- no difference between the response of the genetically modified fly and the response of the wild type fly is indicative of the absence of the pheromone in the environment.

In an embodiment, the invention relates to the use as defined above, wherein the response of the genetically modified fly, and possibly the reference response, is analyzed by an artificial intelligence.

In an embodiment, the invention relates to the use as defined above, wherein the genetically modified fly is infertile.

Such an embodiment is environment friendly since it prevents any spreading of the genetically modified flies in the nature, in the case where some of them escape from their containing device.

In an embodiment, the invention relates to the use as defined above, wherein the genetically modified fly has been rendered infertile through incorporation of the *ovoD1* dominant allele that confers female sterility.

In an embodiment, the invention relates to the use as defined above, wherein more than one genetically modified fly are used.

All the embodiments previously described for the use of a genetically modified fly apply *mutatis mutandis* to the other aspects of the invention, namely a method for detecting the presence or absence of a pheromone in an environment and a biosensor for the detection of a pheromone in an environment.

Another aspect of the invention concerns a method for detecting the presence or absence of a pheromone in an environment, comprising:
- a step of exposing to the environment a genetically modified fly expressing a heterologous pheromone receptor, and
- a step of measuring the response of the genetically modified fly.

Another aspect of the invention concerns a biosensor for the detection of a pheromone in an environment, said biosensor comprising at least a genetically modified fly expressing a heterologous receptor of the pheromone.

As used herein, the term "biosensor" designates any device comprising a genetically modified fly as defined above and some means allowing the fly to be in contact to an environment and permitting the detection of its response when the fly is exposed to said environment. Such a biosensor can comprise more than one genetically modified fly. It can also comprise one or more wild type flies, which constitute a control.

In an embodiment, the invention relates to a biosensor as defined above, wherein the biosensor further comprises an electroantennography (EAG) device for recording the electrical signal in the antenna of the genetically modified fly.

In an embodiment, the invention relates to a biosensor as defined above, wherein the biosensor further comprises a device for monitoring the locomotor activity of the genetically modified fly, in particular a device emitting at least an infrared beam or a video camera.

In an embodiment, the invention relates to a biosensor as defined above, wherein the biosensor further comprises a wild type fly.

In an embodiment, the invention relates to a biosensor as defined above, wherein the biosensor comprises more than one genetically modified fly, and possibly more than one wild type fly.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

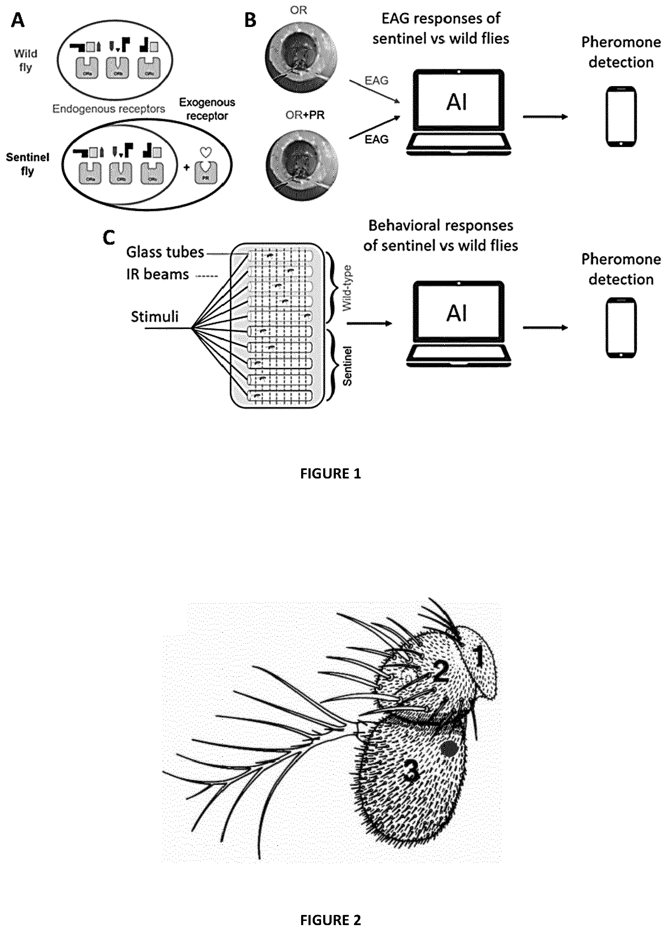

Figure 1. Monitoring the responses to odor stimuli. (A) Sentinel flies express a pheromone sensitive receptor in Orco neurons in addition to endogenous receptors. Responses to odor stimuli can be recorded with EAG (B) or actigraphy (C).
**Figure 2****.** Frontal view of a fly's right antenna. The big black dot indicates the optimal position of the recording electrode. Numbers 1, 2 and 3 indicate the three antennal segments.

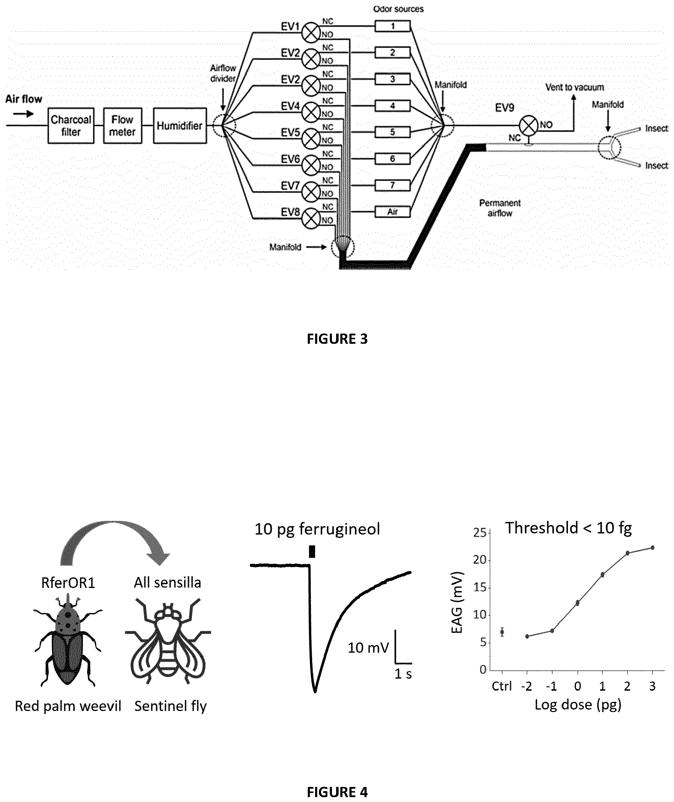

**Figure 3****.** Odor delivery device used to generate training data as responses to pheromone pulses of various durations. An electrovalve upstream of odor sources (any of EV1- EV8) odorizes the passing airflow (200 ml/min in each channel). The downstream electrovalve (EV9) controls the timing of stimuli that are delivered within an aluminum tube (4 mm internal diameter). Odor puffs are transported by a permanent flow of clean air (1400-1600 ml/min) and are delivered to two flies in parallel, a sentinel and a wild type. EV: electrovalves. NO: normally open position of the valve (used when no stimuli are delivered to the insects). NC: normally close position of the valve (used to deliver stimuli to the insects).
**Figure 4****.** Sentinel flies expressing RferOR1 are extremely sensitive to ferrugineol. Responses to ferrugineol were measured by EAG. Dose of pheromone stimuli are 1/10,000 of the load in the source. It was determined according to the diameter of the odor delivery system (7 mm of internal diameter) compared to the size of the antenna (≈ 150 µm of length) and to the number of stimuli the system can provide without any reduction in the amplitude of the EAG response (> 1000). The detection threshold is estimated to be less than 10 fg. Ctrl = hexane (solvent of ferrugineol).

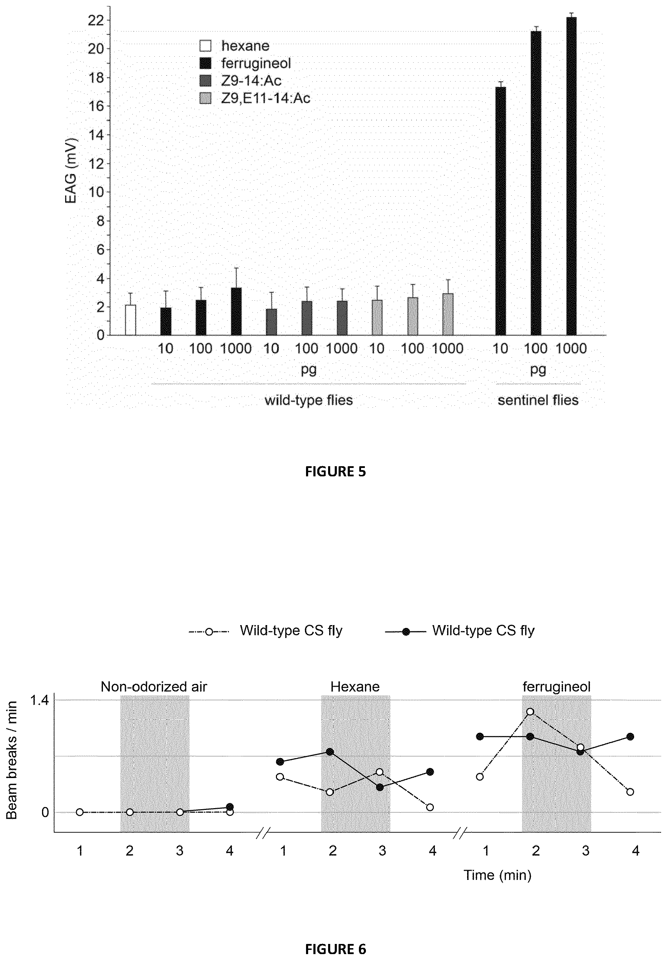

**Figure 5****.** Wild-type flies are not sensitive to the main pheromone components of *S. frugiperda* (Z9-14:Ac), *S*. *littoralis* (Z9,E11-14:Ac) and *Rhynchophorus ferrugineus* (ferrugineol) (one way Anova), while sentinel flies expressing RferOR1 exhibited strong responses to ferrugineol.
**Figure 6****.** Behavioral experiments show RferOR1-expressing sentinel flies are keen detector of ferrugineol. Arousal responses to ferrugineol were measured by actigraphy. Odor was applied for 2 minutes and the resulting behavioral response is captured in the shaded block. The 1^{st} and 4^{th} minute values represent basal activity before and after odor delivery. N = 14 flies.

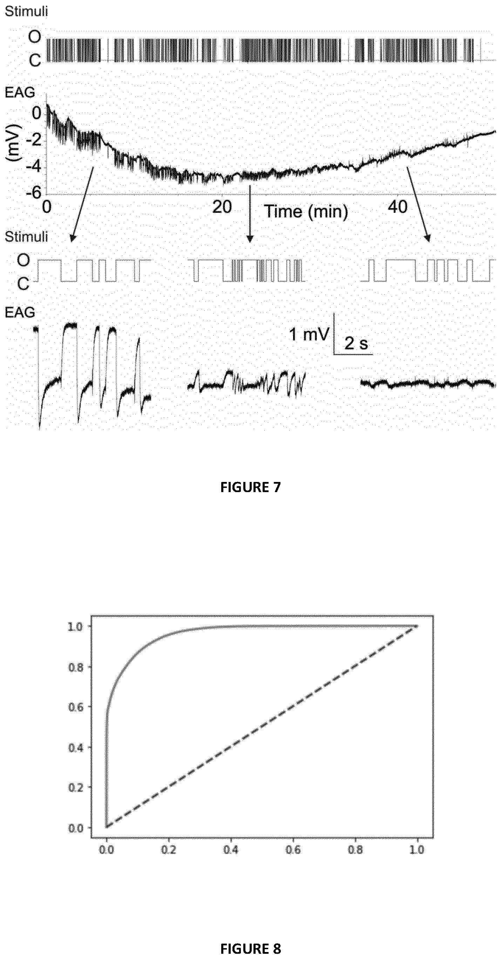

**Figure 7****.** Example of training signal produced by the experimental setup of Figure 3. The class label is the binary signal of the electrovalve (EV9 in Figure 3). C and O correspond to the valve being closed or open and thus to no stimuli or pheromone stimuli, respectively. The classifier input is the EAG signal ranging from 0.1 mV to 10 mV depending on the pheromone dose. The three bottom panels correspond to responses to high (left), intermediate (center) and low pheromone concentration (right).
**Figure 8****.** Receiver Operating Curve (ROC) for the classifier trained on EAG data. The ROC represents the detection rate (true positive) vs. the false alarm rate (false positive).

### EXAMPLES

The sensors of the present invention were engineered from insect pheromone receptors (PRs), the key actors of insects' unmatched olfactory performances, to monitor endemic or potentially invasive insect pests. To this end, PRs of the insect pest were expressed in most (≈80%) of the olfactory receptor neurons (ORNs) of a *Drosophila.* This so-called "sentinel" fly becomes ultra-sensitive to the pest pheromone, unlike a wild fly which is not. Pheromone selectivity was thus made possible by analyzing, in particular via machine learning, the responses of sentinel and wild flies (**Figure 1**).

This sensor technology was developed and tested for the detection of the three major components of the pheromones of three major insect pests: 4-methylnonan-5-ol (= ferrugineol) for *Rhynchophorus ferrugineus* (Coleoptera), (Z)-9-tetradecenyl acetate (Z9-14:Ac) for *Spodoptera frugiperda* (Lepidoptera) and (Z,E)-9,11-tetradecadienyl acetate (Z9,E11-14:Ac) for *Spodoptera littoralis* (Lepidoptera).

### Materials & methods

### Engineering of sentinel flies

The open reading frame (ORF) of the *RferOR1* gene of *R. ferrugineus,* the ORF of the SIitORS of S. *littoralis* or the ORF of *Sfru*OR13 of *S*. *frugiperda* is cloned into the pUAST.attB vector, which is subsequently injected into *Drosophila* embryos expressing PhiC31 integrase and harboring an attP landing site at the ZH-51C locus of chromosome 2R, using the method described in Antony et al., Mol. Ecol. 30(9):2025-2039, 2021.

In addition, this strategy was modified to further boost the magnitude of sentinel fly's EAG responses, and gain more specificity.
- Boost of the magnitude of sentinel fly's EAG responses is accomplished by:
   - High-expression UAS: pJFRC81-based 10XUAS-IVS-Syn21-ORx-p10 vectors containing 10x copies of the UAS are used to maximize the binding of the transactivator Gal4, with an intronic IVS that promotes ORx mRNA export to the cytoplasm, a synthetic translation enhancer *Syn21* in the 5' UTR and a p10 terminator in the 3' UTR to increase ORx mRNA stability.
   - Achieving broader GAL4 expression: the odorant co-receptor (Orco) is at best present in 70-80% of the antennal OSNs. Indeed, about a quarter of the ORNs express ionotropic receptors (IRs), an altogether different family of chemoreceptors. To express the pest PR in all ORNs of the sentinel fly a second transgene is added: *pebbled*-gal4 which drives expression in both Orco(+) and IR(+) ORNs. The two driver transgenes Orco-GAL4 and pebbled-GAL4 work side-by-side. It leads to an increase in the EAG amplitude due to wider and higher level of PR protein expression.

- Gain of specificity of sentinel fly's EAG responses is accomplished by the use of:
   - Two different UAS-ORx lines in two separate flies, tested simultaneously to overcome ligand promiscuity and receptor promiscuity. Most of the PRs (e.g., SIitORS) indeed respond specifically to a single pheromone compound of the targeted species. However, this compound could also be widely shared by non-targeted species in their respective pheromone blends. On the other hand, some PRs (e.g., SfruOR13) non-specifically respond to pheromone component of non-targeted species and/or plant volatiles in addition to robustly responding to the major sex pheromone component of the targeted species' female. To avoid false positives, both the major and the minor component of the sex pheromone blend of the targeted species are searched in the field in parallel, using two distinct lines of sentinel flies expressing two different UAS-ORx lines. Artificial intelligence (Al) is used to decode pheromone information from the behavioral response of the two sentinel flies and a third group of wild-type flies. The double-check dramatically increases specificity.

### Measuring pheromone responses from sentinel flies

The responses of sentinel flies to pheromone stimuli are monitored with two different techniques:
(i) the measure of electrical responses of their antennae by electroantennography (EAG) or (ii) the measure of their behavioral responses with actigraphy

With both techniques, EAG and actigraphy, the selectivity of pheromone detection is made possible by machine learning on the responses of sentinel and wild flies. The responses observed in sentinel flies but not in wild flies thus corresponded to pheromone responses.

### i) Electroantennography (EAG)

EAGs are recorded from 2- to 5-day-old adult flies according to standard procedures (Alcorta E., J. Neurophysiol., 65(3):702-714, 1991; Ayer et al., J Neurobiol., 23(8):965-982, 1992). Flies are held in a 100-µl pipette tip whose extremity is cut such that the anterior portion of the fly's head just protrudes, leaving the antennae exposed. Two glass electrodes (tip diameter 1-2 µm) are filled with 120 mM NaCl, 5 mM KCI, 1 mM CaCl₂, 4 mM MgCl₂, and 10 mM HEPES. The electrodes are in contact with a high impedance differential amplifier via silver/silver-chloride wires. The reference electrode is inserted in one eye and the recording electrode is pressed against the third antennal segment of one antenna in median position (Figure 2) using manual micromanipulator (NMN-25 Narishige). The signal is amplified (×100), low-pass filtered (1 kHz) and digitized at 1 kHz.

Odor stimuli are obtained by blowing an air puff through a pipette or vial containing a known quantity of a given odorant and responses are expressed according to stimulus load (Figures 4, 5).

### ii) Behavioral experiments

Lab cultured flies fed with a special 4-24R diet that increases their resilience are collected under mild CO₂ anesthesia using Zeiss' stemi-508-M stereomicroscope. The flies are subsequently introduced into custom-made behavioral apparatus.

DAM5H monitors of TriKinetics using 15 infrared beams detect the movement of flies in 80-mm tubes, and transmit the number of beam-breaks to a computer through the LC4 acquisition board. The 80-mm long tubes into linear olfactometers were transformed by fitting a digitally controlled air-delivery tube on the front end and odor-extraction tube on the other end. Precise odor delivery is accomplished by manifolds regulated via 3-way electrovalves, whose opening/closing is directed by the same acquisition board that interfaces with DAM5H. In preliminary experiments on sentinel flies (RferOR1-flies), kinesis (non-directed movement), taxis (directed movement) and dwell (preference for a specific location) behaviors induced by odor were measured. Any of these behavioral modules in field conditions, is a reliable indicator of the presence of the pest insect in the environment.

### Machine learning

For the EAG-based sensor, a classifier is trained with supervised learning on labeled responses of both sentinel and wild flies. The training set is produced in the lab as responses to pheromone pulses of various durations using an experimental setup like the one depicted in Figure 3.

### Example 1. Detection of ferrugineol by sentinel fly's EAG

Sentinel flies expressing the PR *Rfer*OR1 of the red palm weevil exhibited robust high-amplitude EAG responses that are clearly distinguishable from the wild *Drosophila's* meagre EAG responses to ferruginol, the palm weevil pheromone component and cognate ligand of *Rfer*OR1 (Figures 4 and 5).

### Example 2. Detection of ferrugineol by sentinel flv's behavior

The obtained results showed that ferrugineol arouses sentinel flies, causing them to move at higher rate during odor exposure, but has no effect on wild-type flies. Sentinel's response readily goes down as pheromone delivery is terminated. Moreover, non-specific odors elicit similar response dynamics in wild-type and sentinel flies. In outdoor experiments under semi-natural conditions, actigraphic data can be registered from the same set of sentinel flies for over a week.

### Example 3. Analysis of the signal by Machine learning

The responses observed in sentinel flies and not in wild type flies corresponded to pheromone responses. In the field, these responses can be very noisy and of low amplitude, hence requiring advanced techniques of AI for detection.

For the EAG-based sensor, a classifier was trained with supervised learning on labeled responses of both sentinel and wild flies. The training set was produced in the lab as responses to pheromone pulses of various durations using an experimental setup like the one depicted in Figure 3. An example of training sequence is shown in Figure 7. The EAG and electrovalve signals provide inputs and class labels for the classifier (Figure 7).

For the actigraphy-based sensor, the classifier input corresponds to the trajectories of sentinel and wild flies in glass tubes provided by the IR beams (Figure 1). After training, classification performance is assessed as Receiver Operating Curve (Figure 8) from an independent dataset.

## Claims

1. Use of a genetically modified fly expressing a heterologous pheromone receptor for detecting the presence or absence of the pheromone in an environment,
the presence or absence of the pheromone being detected by measuring the response of the genetically modified fly exposed to the environment.

2. Use according to claim 1, wherein the pheromone is from an insect pest.

3. Use according to claim 1 or 2, wherein the insect pest is chosen from the group comprising coleoptera, diptera, hemiptera, hymenoptera, lepidoptera, neuroptera, orthoptera or thysanopetera.

4. Use according to any one of claims 1 to 3, wherein the insect pest is chosen from the group comprising *Spodoptera frugiperda, Spodoptera littoralis* or *Rhynchophorus ferrugineus.*

5. Use according to any one of claims 1 to 4, wherein the heterologous pheromone receptor is expressed in the majority of the fly's olfactory receptor neurons.

6. Use according to any one of claims 1 to 5, wherein the response of the genetically modified fly is an electrical signal in its antenna.

7. Use according to any one of claims 1 to 5, wherein the response of the genetically modified fly is its locomotor activity.

8. Use according to any one of claims 1 to 7, wherein the response of the genetically modified fly is compared to a reference response.

9. Use according to any one of claims 1 to 8, wherein the response of the genetically modified fly is compared to the response of a wild type fly exposed to the environment,
a difference between the response of the genetically modified fly and the response of the wild type fly being indicative of the presence of the pheromone in the environment, and no difference between the response of the genetically modified fly and the response of the wild type fly is indicative of the absence of the pheromone in the environment.

10. Use according to any one of claims 1 to 9, wherein the response of the genetically modified fly is analyzed by an artificial intelligence.

11. A method for detecting the presence or absence of a pheromone in an environment, comprising:
- a step of exposing to the environment a genetically modified fly expressing a heterologous pheromone receptor, and
- a step of measuring the response of the genetically modified fly.

12. Biosensor for the detection of a pheromone in an environment, said biosensor comprising at least a genetically modified fly expressing a heterologous receptor of the pheromone.

13. Biosensor according to claim 12, wherein the biosensor further comprises an electroantennography (EAG) device for recording the electric signal in the antenna of the genetically modified fly.

14. Biosensor according to claim 13, wherein the biosensor further comprises a device for monitoring the locomotor activity of the genetically modified fly, in particular a device emitting at least an infrared beam or a video camera.

15. Biosensor according to claim 14, wherein the biosensor further comprises a wild type fly.
